# EUROPEAN PATENT SPECIFICATION

(11) **EP 0 409 511 B1**
(45) Date of publication and mention of the grant of the patent: **09.03.1994**
(21) Application number: 90307717.0
(22) Date of filing: 13.07.1990
(51) Int. Cl.: A61M 5/168, G05D 7/01

(54) **Adjustable flow regulator for use in an implantable drug infusion system**
Einstellbarer Durchflussregler für ein implantierbares Medikamenteninfusionssystem
Régulateur de débit réglable pour un système de perfusion implantable

(30) Priority: 18.07.1989 US 381350
(43) Date of publication of application: 23.01.1991
(73) Proprietor: INFUSAID INC., Norwood, Massachusetts (US)
(72) Inventor: Sampson, Edward J., Carlisle, Massachusetts (US)
(74) Representative: Bradbrook, Geoffrey William

(56) References cited:
- WO-A-80/02377
- FR-A- 1 299 719
- US-A- 3 731 681
- US-A- 4 241 757

## Description

This invention is directed to an adjustable flow regulator for use in an implantable drug delivery system for accurately controlling the flow rate of a drug. This invention is an improvement which relates to a compensating mechanism for an implantable pump. This device is used to deliver drugs at a very slow rate over a long period of time between subcutaneous refills.

An implantable infusion pump of the prior art (US-A-3,731,681) utilizes the vapor pressure of a two stage gas to maintain a constant pressure on a drug flowing through a capillary tube in order to maintain a constant flow rate. This technique of flow control while simple and reliable is sensitive to outside variables such as body temperature and atmospheric pressure.

Another implantable pump system of the prior art (US-A-4,299,220 or WO-A-8 002 377) employs a regulator to compensate for variations in pressure and temperature to provide a more accurate and uniform rate of drug delivery. This regulator employs a body having a shallow internal cavity and a flexible diaphragm in the body which divides the cavity into two sections. An outlet from the second of the sections is centrally disposed in the wall of the cavity underlying the diaphragm. Flexing of the diaphragm in one direction contacts an elastomeric sealing ring around the outlet and closes the fluid passage-way. The inlet of the regulator body is connected to the capillary flow line from a pressure actuated drug delivery device. The flow line includes a capillary restrictor upstream from the inlet to the second section. The capillary restrictor is thus in series with the flow control valve which is formed by the outlet and the diaphragm. When the opposing forces on the diaphragm are stable, the diaphragm is stationary. If there is a change in these forces, the diaphragm deflects either to close the valve when the pressure difference is negative in the second section or to open the valve if the pressure difference is positive in the second section. One of the difficulties in this system is the ability to adjust the position of the diaphragm in relation to the valve. Consequently, the device which is generally a fixed assembly provides a widely varying initial flow rate due to manufacturing tolerances and is impossible to normalize.

It is an object of the invention to provide an implantable infusion system which can be adjusted and calibrated to insure that performance can be optimized to design specifications and patient requirements.

According to the present invention, there is provided an implantable drug delivery system which corresponds to the definition given in claim 1.

The invention and advantages of the invention will become more apparent from the following description taken in conjunction with the accompanying drawings wherein:
Fig. 1 is a schematic representation of the adjustable flow regulator according to this invention when used in conjunction with a pressure actuated rug delivery system; and
Fig. 2 is a section view of the flow regulator as illustrated in Fig. 1.

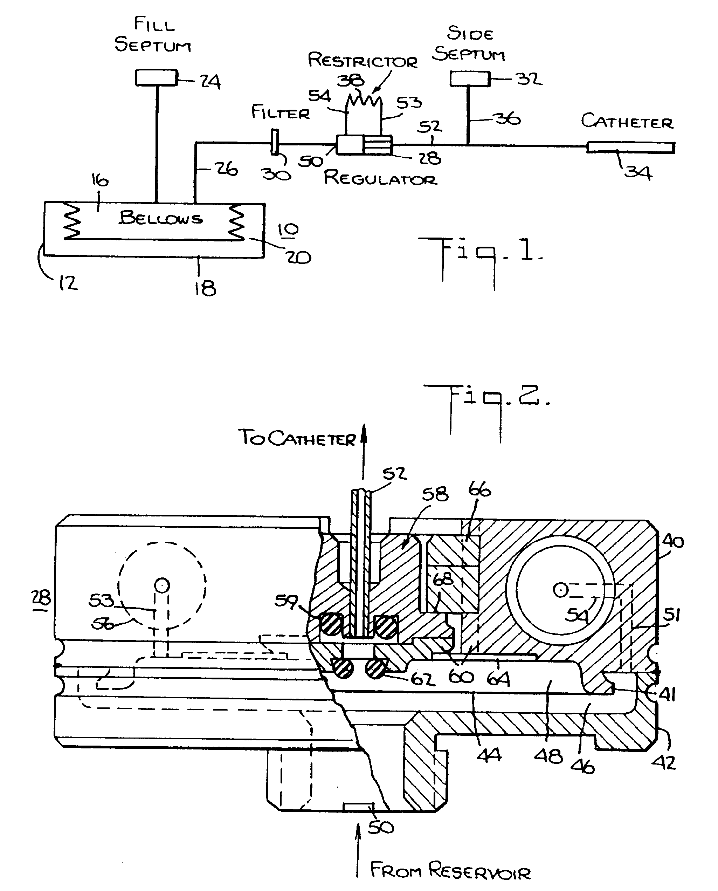

The implantable drug delivery system of the present invention is illustrated schematically in Fig. 1. It comprises an infusion pump 10 with a housing 12 divided into a drug chamber 16 and a propellant chamber 18 by means of a bellows or diaphragm 20. The infusion pump is implanted under the skin and the drug chamber may be refilled hypodermically utilizing a penetrable resilient fill septum 24. The chamber 18 contains "Freon" having a vapor pressure such that, under conditions of normal body temperature, creates a pressure upon the bellows 20 to force a drug contained in the chamber 16 out through the discharge opening 26, through a filter 30, to an adjustable flow rate regulator 28. The regulator incorporates a capillary type restrictor 38.

Referring to Fig. 2, the flow regulator 28 comprises a body formed by a top member 40 and a bottom member 42. The upper portion of the bottom member 42 and the lower portion of the top member 40 form an interior cavity and have mating surfaces at their peripheries. The mating peripheral surfaces of the top member 40 and the bottom member 42 are assembled and fastened together by any convenient means such as screws, (not illustrated) welding or the like. A resilient diaphragm 44 is welded to an annular flange 41 extending from the top member 40. The diaphragm is composed of a flexible but impervious material, such as titanium and is disposed in the cavity which the diaphragm divides into a lower section 46 and an upper section 48. The bottom member 42 has an inlet 50 which communicates with section 46. An outlet 52 communicates with section 48 and connects the regulator to the catheter 34.

Fluid from the drug chamber 16 flows through inlet 50 into the lower section 46. Fluid from the lower section 46 is delivered to the upper section 48 via duct 51 and opening 54 which are interconnected to opening 56 and duct 53 via the restrictor 38. By this technique then, fluid is delivered unrestricted to section 46 and then through the restrictor 38 to section 48. Thus, the restrictor 38 communicates directly across the diaphragm 44.

The outlet 52 is welded to an adjustable fitting 58 within top member 40. An O-ring 59 provides a seal in a recess of the fitting. The inner surface of the adjustable fitting engages a sealed movable annular plate 60. The plate 60 has a flexible metallic seal 64 welded at its periphery in a narrow recess in the top member 40 and has an O-ring valve 62 near its center. As can be appreciated then, adjustment of the fitting 58, produces deflection in the plate 60 and O-ring valve 62. Adjustment is by means of an internal Castle nut 66. By rotation of the Castle nut 66, which bears on a flange 68 of the upper adjustable fitting 58, a downward pressure is exerted on plate 60 and the O-ring valve 62 is urged towards the diaphragm 44. When the O-ring valve contacts the diaphragm the outlet 52 is thereby effectively sealed off. Adjustment from that sealed position is a function of movement of the Castle nut 66 so that a clearance exists between the O-ring valve and the surface of the diaphragm. By this technique, effective attenuation of the flow from section 48 to the outlet 52 can be established and maintained automatically by the varying forces on the diaphragm 44 due to changes in body temperature and atmospheric pressure.

This technique also provides for adjustment during manufacture to insure that the device is properly calibrated. Without such adjustment, the operation of the device would be a function of manufacturing tolerances which provide an inadequate basis by which to insure a predetermined regulated flow given the low volume flow rates.

In operation, medication from the drug chamber 16 is forced through the flow line 26 by the constant pressure exerted by the material in the chamber 18. The medication passing through the filter 30 is them delivered to the compensator through inlet 50. Medication flows into lower section 46 and via opening 54 into the restrictor 38. Then, it is delivered to the upper section 48 and through the outlet 52 into the catheter 34. Given the fact that there is fluid in both sections 46 and 48, opposing forces on the diaphragm generally null the system so that the diaphragm tends to remain stationery.

If, however, there is a change in these forces, for example, as a result of a decrease in flow through the catheter 34 because of a higher atmospheric pressure, then the diaphragm 44 will be deflected downward, due to a build up of pressure in section 48, automatically opening valve 62 increasing flow. If, on the other hand, the pressure in section 48 is reduced by a lowering a atmospheric pressure such as at a higher altitude, the diaphragm will be deflected upward toward O-ring valve 62 automatically closing the valve and reducing flow. Thus, the position of the diaphragm, which is controlled by the pressure differential between sections 46 and 48, effectively maintains a constant or near constant flow rate through outlet 52 to catheter 34.

Given the fact that movement of the diaphragm 44 to establish a flow rate from a fully seated no flow rate condition to the desired dosage level is very small, as discussed herein adjustability of the system is required. Thus, by using the Castle nut 66 and the adjustable diaphragm feature of movable plate 60 the initial distance between the O-ring 62 valve and the diaphragm 44 can be adjusted and set to give proper flow rate. This is a considered to be a significant improvement over prior art systems which do not allow for such adjustability.

It is apparent that modification and variations of this invention may be made without departing from the essential scope thereof, as defined by the following claims.

## Claims

1. An implantable drug delivery system comprising a pressure actuated drug delivery device (10) having a housing (12), a movable bellows (20) dividing said housing into a first chamber (16) containing a drug to be dispensed and a second chamber (18) containing a material exerting pressure on said bellows, means (24) for providing access to said first chamber for refilling it with a drug, a flow regulator (28) connected to said first chamber, said flow regulator comprising a body (40,42), an inlet (50) in said body communicating with said first chamber and an outlet (52) in said body communicating with a catheter (34), a cavity divided by a flexible diaphragm (44) to define a first section (46) and a second section (48) with one section being disposed on each side of said diaphragm, means establishing fluid communication between said two sections, with the first section (46) being in fluid communication with said inlet (50) and with the second section (48) being in fluid communication with said outlet, characterized in that said outlet (52) is movable with respect to said body (40,42) and to said diaphragm (44), and in that means (66) are provided within the regulator (28) for moving said outlet with respect to said diaphragm for adjusting the flow rate through said outlet, whereby said flow regulator is adjustable to be set at a predetermined rate.

2. The drug delivery system of claim 1 further characterized by an adjustable fitting (58) sealed within said body and having a flange (68), said outlet connected to said adjustable fitting, a movable plate (60) coupled to said adjustable fitting and flexibly sealed at the periphery to said body allowing only movement substantially perpendicular to said diaphragm (44), said plate including an O-ring valve (62) engageable with said diaphragm, and wherein said movement means comprises an adjusting nut (66) mounted in said body and contacting said flange on said adjustable fitting, whereby movement of said nut positions said fitting and said O-ring valve relative to said diaphragm.

3. The drug delivery system of claim 1, wherein said diaphragm (44) is of a resilient material mounted in said body, said diaphragm being movable in response to a pressure differential between said first section (46) and said second section (48).

4. The drug delivery system of claim 2 further comprising a flexible metallic seal (64) mounted to the periphery of said movable plate (60) and to said body, said seal being movable in response to movement of said adjusting nut (66) to maintain a seal in the other of said sections.

5. The drug delivery system of claim 1, wherein said regulator body comprises a bottom member (42) having said inlet (50) and a recessed portion defining said first section (46), and a top member (40) having said outlet (52) and restrictor means (38) for providing fluid communication between said sections, and means for joining said bottom member and said top member together in a sealed manner.

6. The drug delivery system of claim 5, wherein said top member (40) includes an annular flange (41) with said diaphragm being mounted on said flange.

## Patentansprüche

1. Implantierbares Arzneimittelverabreichungssystem, umfassend eine druckbetätigte Arzneimittelverabreichungsvorrichtung (10) mit einem Gehäuse (12), einem beweglichen Balg (20), der das besagte Gehäuse unterteilt in eine erste Kammer (16), die ein zu verabreichendes Arzneimittel enthält, und eine zweite Kammer (18), die ein Material enthält, das einen Druck auf den besagten Balg ausübt, Mittel (24) zum Schaffen eines Zugangs zu der besagten ersten Kammer, um sie wieder mit einem Arzneimittel aufzufüllen, einen mit der besagten ersten Kammer verbundenen Durchflußmengenregler (28), wobei der besagte Durchflußmengenregler umfaßt: einen Körper (40, 42), einen mit der besagten ersten Kammer verbundenen Einlaß (50) in dem besagten Körper und einen mit einem Katheter (34) verbundenen Auslaß (52) in dem besagten Körper, eine Ausnehmung, die zur Abgrenzung eines ersten Abschnitts (46) und eines zweiten Abschnitts (48) von einer flexiblen Membran (44) unterteilt wird, wobei ein Abschnitt auf jeder Seite der besagten Membran angeordnet ist, Mittel, die eine Fluidverbindung zwischen den besagten zwei Abschnitten etablieren, wobei der erste Abschnitt (46) in Fluidverbindung mit dem besagten Einlaß (50) steht, und wobei der zweite Abschnitt (48) in Fluidverbindung mit dem besagten Auslaß steht, dadurch gekennzeichnet, daß der besagte Auslaß (52) in Bezug zu dem besagten Körper (40, 42) und der besagten Membran (44) bewegbar ist, und daß Mittel (66) innerhalb des Reglers (28) vorgesehen sind, um den besagten Auslaß in Bezug zu der besagten Membran zu bewegen, um die Durchflußrate durch den besagten Auslaß zu verstellen, wodurch der besagte Durchflußmengenregler verstellbar ist, so daß er bei einer vorbestimmten Rate eingestellt werden kann.

2. Arzneimittelverabreichungssystem nach Anspruch 1, weiter gekennzeichnet durch einen verstellbaren Anschluß (58), der innerhalb des besagten Körpers abgedichtet ist, und einen Flansch (68) aufweist, wobei der besagte Auslaß mit dem besagten verstellbaren Anschluß verbunden ist, eine bewegliche Platte (60), die mit dem besagten verstellbaren Anschluß verbunden und flexibel am Umfang dicht mit dem besagten Körper verbunden ist, so daß nur eine Bewegung im wesentlichen senkrecht zu der besagten Membran (44) möglich ist, wobei die besagte Platte ein O-Ringventil (62) einschließt, das mit der besagten Membran in Eingriff treten kann, und bei welchem die besagte Bewegungsvorrichtung eine Verstellmutter (66) umfaßt, die in dem besagten Körper angebracht ist und den besagten Flansch auf dem besagten verstellbaren Anschluß berührt, wodurch eine Bewegung der besagten Mutter den besagten Anschluß und das besagte O-Ringventil relativ zu der besagten Membran positionieren.

3. Arzneimittelverabreichungssystem nach Anspruch 1, bei welchem die besagte Membran (44) aus einem elastischen Material besteht, das in dem besagten Körper angebracht ist, wobei die besagte Membran als Reaktion auf eine Druckdifferenz zwischen dem besagten ersten Abschnitt (46) und dem besagten zweiten Abschnitt (48) beweglich ist.

4. Arzneimittelverabreichungssystem nach Anspruch 2, weiter umfassend eine am Umfang der besagten beweglichen Platte (60) und an dem besagten Körper angebrachte flexible metallische Dichtung (64), wobei die besagte Dichtung als Reaktion auf eine Bewegung der besagten Verstellmutter (66) beweglich ist, um eine Abdichtung in dem anderen der besagten Abschnitte aufrechtzuerhalten.

5. Arzneimittelverabreichungssystem nach Anspruch 1, bei welchem der besagte Reglerkörper umfaßt: ein Unterteil (42) mit dem besagten Einlaß (50) und einem mit einer Ausnehmung versehenen Teil, welche den besagten ersten Abschnitt (46) bildet, sowie ein Oberteil (40) mit dem besagten Auslaß (52) und einer Drosselvorrichtung (38), um eine Fluidverbindung zwischen den besagten Abschnitten bereitzustellen, und Mittel, um das besagte Unterteil und das besagte Oberteil in einer abgedichteten Weise miteinander zu verbinden.

6. Arzneimittelverabreichungssystem nach Anspruch 5, bei welchem das besagte Oberteil (40) einen ringförmigen Flansch (41) einschließt, wobei die besagte Membran auf dem besagten Flansch angebracht ist.

## Revendications

1. Système implantable de distribution d'un médicament comportant un dispositif (10) de distribution de médicament actionné par pression ayant une enceinte (12), un soufflet mobile (20) divisé dans ladite enceinte en une première chambre (16) contenant un médicament devant être distribué et une seconde chambre (18) contenant une matière exerçant une pression sur ledit soufflet, un moyen (24) pour établir un accès à ladite première chambre pour qu'elle soit rechargée d'un médicament, un régulateur (28) d'écoulement raccordé à ladite première chambre, ledit régulateur d'écoulement comportant un corps (40, 42), une entrée (50) dans ledit corps communiquant avec ladite première chambre et une sortie (52) dans ledit corps communiquant avec un cathéter (34), une cavité divisée par un diaphragme flexible (44) pour définir une première section (46) et une seconde section (48), une section étant disposée sur chaque côté dudit diaphgrame, des moyens établissant une communication de fluide entre lesdites deux sections, la première section (46) étant en communication de fluide avec ladite entrée (50) et la seconde section (48) étant en communication de fluide avec ladite sortie, caractérisé en ce que ladite sortie (52) est mobile par rapport audit corps (40, 42) et audit diaphragme (44), et en ce que des moyens (66) sont prévus à l'intérieur du régulateur (28) pour déplacer ladite sortie par rapport audit diaphragme afin de régler le débit d'écoulement à travers ladite sortie, grâce à quoi ledit régulateur d'écoulement est réglable pour être positionné à un débit prédéterminé.

2. Système de distribution de médicament selon la revendication 1, caractérisé en outre par un raccord réglable (58) monté de façon étanche dans ledit corps et ayant une bride (68), ladite sortie étant reliée audit raccord réglable, une plaque mobile (60) étant accouplée audit raccord réglable et montée de façon flexible et étanche à la périphérie dudit corps, ne permettant qu'un mouvement sensiblement perpendiculaire audit diaphragme (44), ladite plaque comprenant un clapet (62) à bague torique pouvant être engagé avec ledit diaphragme, lesdits moyens de déplacement comprenant un écrou (66) de réglage monté dans ledit corps et en contact avec ladite bride sur ledit raccord réglable, de manière qu'un mouvement dudit écrou positionne ledit raccord et ledit clapet à bague torique par rapport audit diaphragme.

3. Système de distribution de médicament selon la revendication 1, dans lequel ledit diaphragme (44) est en une matière élastique montée dans ledit corps, ledit diaphragme étant mobile en réponse à une différence de pression entre ladite première section (46) et ladite seconde section (48).

4. Système de distribution de médicament selon la revendication 2, comportant en outre un joint métallique flexible (64) d'étanchéité monté sur la périphérie de ladite plaque mobile (60) et sur ledit corps, ledit joint d'étanchéité étant mobile en réponse à un mouvement dudit écrou (66) de réglage pour maintenir un joint d'étanchéité dans l'autre desdites sections.

5. Système de distribution de médicament selon la revendication 1, dans lequel ledit corps du régulateur comporte un élément de fond (42) ayant ladite entrée (50) et une partie évidée définissant ladite première section (46), et un élément de dessus (40) ayant ladite sortie (52) et un moyen d'étranglement (38) pour établir une communication de fluide entre lesdites sections, et des moyens pour joindre entre eux ledit élément de fond et ledit élément de dessus de manière étanche.

6. Système de distribution de médicament selon la revendication 5, dans lequel ledit élément de dessus (40) comprend un rebord annulaire (41), ledit diaphragme étant monté sur ledit rebord.
